# EUROPÄISCHE PATENTSCHRIFT

(11) **EP 1 251 801 B1**
(45) Veröffentlichungstag und Bekanntmachung des Hinweises auf die Patenterteilung: **09.09.2009**
(21) Anmeldenummer: 01900600.6
(22) Anmeldetag: 26.01.2001
(51) Int. Cl.: A61F 2/16

(54) **LINSENIMPLANTAT**
LENS IMPLANT
IMPLANT CRISTALLINIEN

(30) Priorität: 03.02.2000 CH 2122000
(43) Veröffentlichungstag der Anmeldung: 30.10.2002
(73) Patentinhaber: ACCOMMO AG, 4102 Binningen (CH)
(72) Erfinder: HAEFLIGER, Eduard, Anton, CH-4057 Basel (CH)
(74) Vertreter: Blum, Rudolf Emil
(86) Internationale Anmeldenummer: PCT/IB2001/000091
(87) Internationale Veröffentlichungsnummer: WO 2001/056508

(56) Entgegenhaltungen:
- EP-A- 0 166 051
- EP-A- 0 337 390
- EP-A- 0 537 643
- WO-A-93/02639
- WO-A-97/26842
- FR-A- 2 698 264
- US-A- 5 275 623

## Beschreibung

Diese Anmeldung beansprucht die Priorität der Schweizer Patentanmeldung 0212/00, die am 3. Februar 2000 eingereicht wurde.

### Hintergrund

Die Erfindung betrifft ein Linsenimplantat und einen Bausatz gemäss Oberbegriff der unabhängigen Ansprüche.

Ein Linsenimplantat dieser Art ist z.B. aus US 4 608 050 oder WO 89/00029 bekannt. Es besteht aus einem flexiblen Körper, der die Linsenmasse des Auges ersetzt. Seine Form und Grösse entspricht im wesentlichen jener einer typischen Linsenmasse. Dank seiner Flexibilität erlaubt es eine gute Akkommodation des Auges.

Um dieses Linsenimplantat den individuellen Anforderungen eines Auges anzupassen, müssen eine Vielzahl von Implantatmassen mit unterschiedlichem Brechungsindex bereitgestellt werden. Dies ist aufwendig. Ausserdem zeigt es sich, dass die Implantatmassen die Anforderungen an Festigkeit und gleichzeitiger Akkommodationsfähigkeit nur beschränkt erfüllen.

FR 2 698 264 beschreibt eine Linse aus zwei Teillinsen, zwischen denen eine Füllmasse angeordnet wird. Die Teillinsen bilden die Aussenform der Linse und ersetzen die Linsenkapsel. Dadurch wird die Anbindung der Linse an den okkularen Akkommodationsmechanismus schwierig. Ausserdem unterscheiden sich die mechanischen Eigenschaften der Linse stark von einer natürlichen Linse, was die Akkommodation erschwert.

In US 5 275 623 wird ein Linsenimplantat gezeigt, welches aus einem Beutel mit zwei Linsen besteht. Der Beutel wird mit einem Gas oder einer Flüssigkeit gefüllt. Diese Linse besitzt aufgrund ihrer Symmetrie jedoch grundlegend von der natürlichen Linse abweichende Eigenschaften, so dass sie in der Praxis kaum zu befriedigen vermag.

### Darstellung der Erfindung

Es stellt sich deshalb die Aufgabe, ein Linsenimplantat bzw. einen Bausatz und ein Verfahren der eingangs genannten Art bereitzustellen, die diesen Nachteil vermeiden.

Diese Aufgabe wird von der Linse bzw. dem Kit und dem Verfahren gemäss den unabhängigen Ansprüchen erfüllt.

Erfindungsgemäss besteht das Linsenimplantat also aus einem ersten, verformbaren Teil und aus einem zweiten Teil, wobei die beiden Teile unterschiedlichen Brechungsindex und/oder unterschiedliche elastische Verformbarkeit aufweisen. Der erste Teil bildet die proximale Oberfläche und der zweite Teil die distale Oberfläche des Linsenimplantats.

Somit kann das Implantat aus zwei unterschiedlichen Teilen zusammengesetzt werden, was es erlaubt, die Brennweite und/oder die mechanischen Eigenschaften den jeweiligen Anforderungen in einfacher Weise anzupassen. So kann z.B. der erste Teil in erster Linie die Grösse bzw. das Volumen der Linse definieren und die Akkommodation gewährleisten, während durch Wahl des zweiten Teils die Brennweite und/oder distale Form festgelegt wird.

Das Implantant weist im wesentlichen die Form der zu ersetzenden Linsenmasse auf, so dass es in der Linsenkapsel Platz findet und diese gut ausfüllt. Hierzu sollte die distale Oberfläche des Implantats stärker gekrümmt sein als die proximale Oberfläche.

Vorzugsweise besteht der erste Teil aus einem Material, welches intraokular von einem fliessfähigen zu einem elastisch verformbaren Zustand aushärtbar ist, so dass er in einfacher Weise der Form der zu ersetzenden Linsenmasse angepasst werden kann.

Für eine gute Akkommodationsfähigkeit sollte der erste Teil besser elastisch verformbar sein als der zweite Teil, insbesondere wenn letzterer die distale Oberfläche des Linsenimplantats definiert.

Der zweite Teil kann elastisch deformierbar ausgestaltet werden, geeignet um in aufgerolltem oder gefaltetem Zustand in die Linsenkapsel eingebracht zu werden. Dies ist besonders vorteilhaft in Kombination mit einem aushärtbaren ersten Teil, da der erste Teil in diesem Fall zum Ausfüllen der Linsenkapsel verwendet werden kann und die Akkommodationsfähigkeit garantiert, während mit dem zweiten Teil die Brennweite des Linsenimplantats festgelegt wird.

Der zweite Teil bildet vorzugsweise den distalen Abschnitt des Linsenimplantats, so dass er eine Wand bildet, die den ersten Teil vom distalen Teil der Linsenkapsel trennt. Dies erlaubt es, nötigenfalls den distalen Teil der Linsenkapsel zu entfernen, wenn sich dieser trüben sollte.

Der Bausatz zum Herstellen solcher Linsen umfasst vorzugsweise mindestens ein fliessfähiges Füllmaterial sowie eine Vielzahl von vorgeformten Linsenteilen zum Bilden des zweiten Teils. Die Linsenteile besitzen unterschiedliche Formen und/oder Brechungsindizes. Somit können mit nur einem Füllmaterial eine Vielzahl von Linsenimplantaten unterschiedlicher Brennweite und Form hergestellt werden.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 einen Schnitt durch ein normales Auge,
Fig. 2 einen Schnitt durch ein Auge mit Linsenimplantat,
Fig. 3 ein mögliches Herstellungsverfahren für das Linsenimplantat und
Fig. 4 einen Schnitt durch ein Auge mit einer alternativen Ausführung des Linsenimplantats.

### Wege zur Ausführung der Erfindung

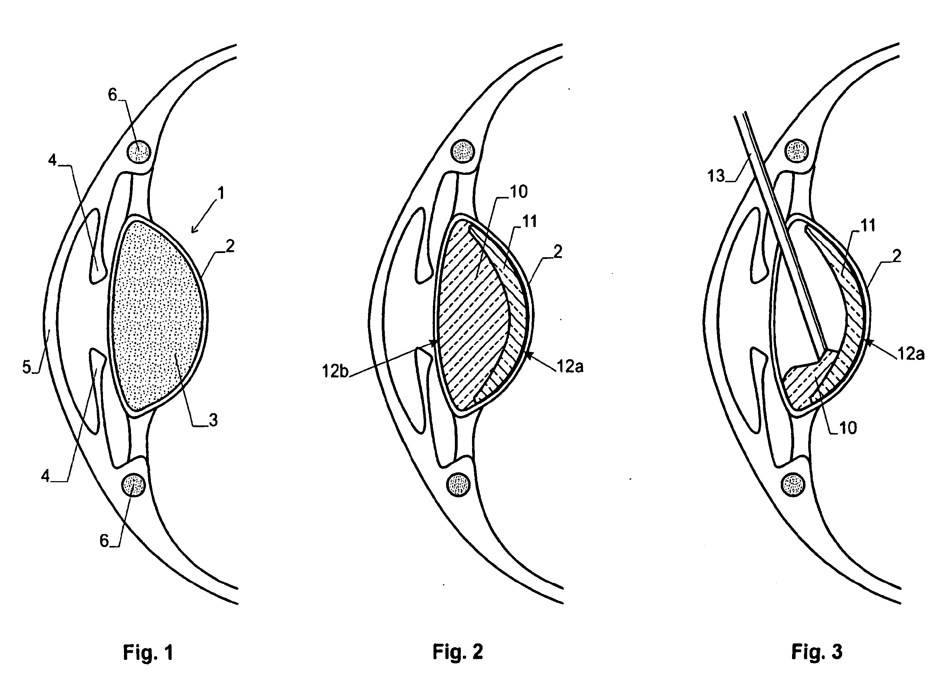

Die im vorliegenden Zusammenhang wichtigsten Teile des Auges sind in Fig. 1 dargestellt und umfassen die Linse 1, bestehend aus Linsenkapsel 2 und Linsenmasse 3, die Iris 4 und die Hornhaut 5.

Die Linse 1 ist ein elastischer Körper, dessen Krümmung sich bei Kontraktion des Müller'schen Muskels 6 ändert, so dass eine Akkommodation des Auges auf unterschiedliche Brennweiten möglich wird. Mit zunehmendem Alter wird die Linsenmasse 3 jedoch härter, was zu einem teilweisen oder vollständigen Verlust der Akkommodationsfähigkeit führen kann.

Die vorliegende Erfindung bezieht sich auf ein Linsenimplantat, das die Linsenmasse 3 des Auges ersetzt. Eine derartige Linse ist in Fig. 2 dargestellt. Sie besteht aus zwei aneinander anliegenden, transparenten Teilen 10, 11, die zusammen etwa die gleiche Grösse und Form haben wie die ersetzte, in die Ferne akkommodierte Linsenmasse 3. Wie bei der Linsenmasse ist also die distale Oberfläche 12a des Linsenimplantants stärker gekrümmt als die proximale Oberfläche 12b. Der Begriff proximal bezeichnet in diesem Zusammenhang die der Hornhaut zugewandte Linsenseite, der Begriff distal die der Retina zugewandte Linsenseite.

Gleiche Grösse und Form wie eine natürliche Linsenmasse besitzt das Linsenimplantat, wenn es eine konvexe proximale Oberfläche mit einem Krümmungsradius zwischen 8.4 und 13.8 mm, eine konvexe distale Oberfläche mit einem Krümmungsradius zwischen 4.6 und 7.5 mm und eine Dicke zwischen 2.8 und 5.5 mm aufweist. Der typische Äquator beträgt um Durchschnitt um die 9 bis 11 mm für erwachsene Augen. Durchschnittliche proximale und distale Krümmungsradien sind 10 bzw. 6 mm.

Das Linsenimplantat, d.h. insbesondere dessen erster Teil, besitzt ähnliche optische und elastische Eigenschaften wie eine Linsenmasse guter Akkommodationsfähigkeit. Es wird im Normalfall von der Linsenkapsel 2 angliegend umschlossen und erzeugt, wie eine natürliche Linsenmasse, einen intrakapsulären Druck. Bei einer Kontraktion des Müller'schen Muskels 6 ändert sich die Krümmung der Oberflächen des Linsenimplantats und somit dessen Brennweite. Das Linsenimplantat verleiht dem Auge also eine natürliche Akkommodationsfähigkeit. Hierzu sollte das Volumen des ersten Teils 10 grösser als jenes des zweiten Teils sein. Vorzugsweise sollte auch der Gesamtdurchmesser senkrecht zur Achse des Auges beim ersten Teil grösser als beim zweiten Teil sein.

Die beiden Teile 10, 11 des Linsenimplantats besitzen vorzugsweise unterschiedliche Brechungsindizes. Der erste, proximale Teil 10 wird bei der Herstellung der Linse der Grösse und Form der zu ersetzenden Linsenmasse 3 angepasst, während der zweite, distale Teil 11 ein vorgefertigter, elastischer Formkörper ist.

Bei der Anfertigung der Linse erfüllen die beiden Teile 10, 11 unterschiedliche Aufgaben. Der proximale Teil 10 bestimmt das Volumen der Linse. Der distale Teil 11 gibt dem hinteren Linsenbereich seine Stabilität und erlaubt es, die Brennweite der Linse dem jeweiligen Auge individuell anzupassen. Hierzu steht dem Fachmann bei der Linsenfertigung ein Bausatz zur Verfügung, der ein fliessfähiges Füllmaterial und mehrere, vorgefertigte Linsenteile umfasst. Die Linsenteile unterschieden sich voneinander durch ihren Brechungsindex und/oder ihre Form.

Das Linsenimplantat wird hergestellt, indem das fliessfähige Füllmaterial und einer der Linsenteile zusammengefügt werden und das Füllmaterial auf die gewünschte Festigkeit ausgehärtet wird. Dies kann ausserhalb des Auges oder intraokular geschehen. Hierzu wird zuerst das Auge ausgemessen, um die Brennweite und Form der Linsenmasse zu ermitteln, bzw. um aus den Augeneigenschaften die gewünschte Brechkraft des Implantats zu bestimmen. Daraus kann berechnet werden, welcher der vorliegenden Linsenteile 11 verwendet werden soll, damit das Linsenimplantat möglichst die gewünschte Brennweite besitzt.

Bei einem bevorzugten Herstellungsverfahren wird das Linsenimplantat intraokular gefertigt. Dabei wird in einem ersten Schritt eine kleine Öffnung in die Linsenkapsel 2 geschnitten, und die Linsenmasse 3 wird entfernt. Dies geschieht vorzugsweise durch Ultraschall, Lasertechnik, Fakoemulsifikation oder Laserfakoemulsifikation. Entsprechende Verfahren sind dem Fachmann bekannt.

In die so ausgehöhlte Linsenkapsel wird sodann der distale Teil 11 der Linse eingeführt. Damit der Linsenteil 11 auch durch eine kleine Öffnung eingebracht werden kann, ist er elastisch deformierbar, so dass er aufgerollt oder gefaltet und mit einem Injektionsinstrument eingeführt werden kann. Ein Gerät dieser Art ist z.B. aus US 5 620 450 bekannt.

Der eingeführte distale Teil 11 entfaltet sich und nimmt in der Linsenkapsel in etwa die gewünschte Position ein. Er ist in entspanntem Zustand konkav-konvex gekrümmt, wie dies in Fig. 2 und 3 dargestellt ist. Er bildet die distale Seite des Linsenimplantats und die Form seiner konvexen Fläche 12a entspricht jener der distalen Oberfläche der zu ersetzenden Linsenmasse. Je nach Anforderung an die optischen und mechanischen Eigenschaften kann der distale Teil 11 auch bikonvex oder plankonvex sein, d.h. seine proximale Oberfläche kann nach vorne oder nach hinten gekrümmt oder flach sein.

Nachdem der distale Linsenteil positioniert ist, wird über ein Injektionsgerät 13 der proximale Linsenteil 10 als Füllmaterial in fliessfähiger Form in die Linsenkapsel 2 eingespritzt, und die Linsenkapsel 2 wird in ihrem auf die Ferne akkommodiertem Zustand gefüllt. Es wird dabei soviel Füllmaterial eingeführt, dass das Volumen des Linsenimplantants im wesentlichen jenem der zuvor entfernten Linsenmasse entspricht.

Danach wird die Linsenkapsel verschlossen und der proximale Linsenteil 10 wird auf die gewünschte Festigkeit ausgehärtet, was z.B. mittels chemisch, elektrisch oder optisch induzierter Vernetzung geschehen kann. Geeignete Füllmaterialien und -techniken sind dem Fachmann z.B. aus US 4 608 050 oder WO 89/00029 bekannt.

Als Füllmaterial kann z.B. ein vernetztes Polysiloxan, ein Hydrogel oder ein Collagen-Präparat verwendet werden. Vorzugsweise wird ein Gel eingesetzt, da dies von seiner Konsistenz ähnlich wie die Linsenmasse ist.

Die Anordnung des vorgefertigten Linsenteils 11 an der distalen Seite der Linse hat den Vorteil, dass er eine relativ stabile Rückwand für die Linse bildet. Kommt es nach der Implantation zu einer Trübung im distalen Bereich der Linsenkapsel 2, so kann die Linsenkapsel 2 in diesem Bereich mittels Laserstrahlen entfernt werden. Der Linsenteil 11 schirmt auch ohne Linsenkapsel 2 den möglicherweise noch fliessfähigen, physiologisch schlechter verträglichen ersten Linsenteil 10 vom Glaskörper des Auges und den übrigen Strukturen des Augeninnern ab. Da die Eröffnung der hinteren Kapsel mit einem Laser nie regelmässig ist, würde das weichere Material des ersten Linsenteils ansonsten nach hinten herausquellen, da die Kapsel unter Druck steht. Dadurch würde die optische Funktion der Linse zerstört. Der Linsenteil 11 verhindert einen solchen Austritt.

Insbesondere wenn jedoch ein stabileres, physiologisch gut verträgliches Material für den ersten Linsenteil 10 verwendet wird, so kann der zweite Linsenteil 11 z.B. auch in der Mitte oder in einem proximalen Bereich des Linsenimplantats angeordnet werden.

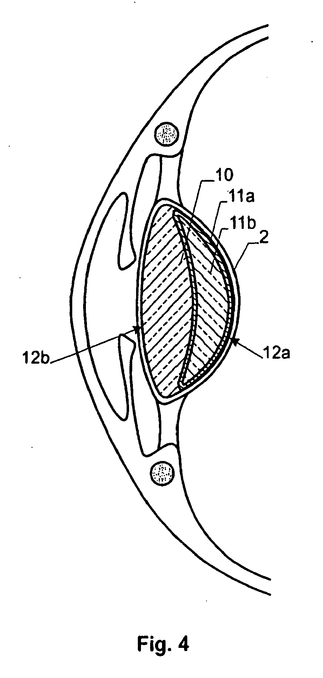

Eine zweite Ausführung der Erfindung wird in Fig. 4 gezeigt. Hier besteht der distale Teil aus einem Beutel 11a, der mit transparenten Füllmaterial 11b gefüllt ist. Dieser Aufbau hat den Vorteil, einen relativ grossen distalen Teil 11a, 11b zu erzeugen, ohne dass ein grosses Loch in die Linsenkapsel gemacht werden muss. Nach dem Entfernen der Linsenmasse 3 wird zuerst der Beutel 11a in leerem, zusammengerollten Zustand in die Linsenkapsel eingebracht. Sodann wird durch die gleiche Öffnung eine Injektionsnadel eingeführt, mit der das Füllmaterial 11b in den Beutel 11a eingeleitet wird. Schliesslich wird der proximale Teil 10 eingefüllt.

Der Beutel 11a ist so geschnitten und vorgeformt, dass er in gefülltem Zustand die gewünschte Form einnimmt. Im oben erwähnten Bausatz kann mindestens ein Teil der vorgeformten Linsenteile, insbesondere die grösseren davon, in Form von Beuteln 11a zur Verfügung gestellt werden.

Gegenstand der vorliegenden Erfindung ist ein Linsenimplantat und ein Bausatz zur Herstellung derartiger Linsenimplantate. Die Erfindung bezieht sich jedoch auch auf das oben beschriebene intraokulare Herstellungsverfahren, bei welchem das Füllmaterial für den ersten Teil 10 der Linse in fliessfähigem Zustand und ein geeigneter Linsenteil 11 in festem Zustand in die Linsenkapsel 2 eingeführt werden. Vorzugsweise wird sodann die Festigkeit des ersten Linsenteils durch Aushärten erhöht. Dabei sollte die Kapsel auf die Ferne akkommodiert sein.

Während in der vorliegenden Anmeldung bevorzugte Ausführungen der Erfindung beschrieben sind, ist klar darauf hinzuweisen, dass die Erfindung nicht auf diese Beschränkt ist und in auch anderer Weise innerhalb des Umfangs der folgenden Ansprüche ausgeführt werden kann.

## Patentansprüche

1. Linsenimplantat zur Anordnung in der Linsenkapsel (2) und zum Ersatz der Linsenmasse (3) eines Auges, welches die Grösse und Form einer natürlichen Linsenmasse aufweist, mit einer distalen Oberfläche (12a) und einer proximalen Oberfläche (12b), wobei die distale Oberfläche (12a) stärker gekrümmt ist als die proximale Oberfläche (12b), und mit einem ersten, verformbaren Teil (10), der von einem fliessfähigen zu einem elastisch verformbaren Zustand aushärtbar ist, und einem zweiten Teil (11), der einen vom ersten Teil (10) unterschiedlichen Brechungsindex und/oder eine vom ersten Teil unterschiedliche elastische Verformbarkeit besitzt, wobei der zweite Teil (11) die distale Oberfläche (12a) bildet, **dadurch gekennzeichnet, dass** der erste Teil (10) die proximale Oberfläche (12b) bildet.

2. Linsenimplantat nach Anspruch 1, **dadurch gekennzeichnet, dass** der zweite Teile einen vom ersten Teil (11) unterschiedlichen Brechungsindex besitzt.

3. Linsenimplantat nach einem der vorangehenden Ansprüche, **dadurch gekennzeichnet, dass** der erste Teil (10) und der zweite Teil (11) aneinander anliegen.

4. Linsenimplantat nach einem der vorangehenden Ansprüche, **dadurch gekennzeichnet, dass** der erste Teil (10) intraokular von einem fliessfähigen zu einem elastisch verformbaren Zustand aushärtbar ist.

5. Linsenimplantat nach einem der vorangehenden Ansprüche, **dadurch gekennzeichnet, dass** der zweite Teil (11) elastisch deformierbar ist zur aufgerollten oder gefalteten Einbringung in die Linsenkapsel.

6. Linsenimplantat nach einem der vorangehenden Ansprüche, **dadurch gekennzeichnet, dass** die Linse aus dem ersten Teil (10) und dem zweiten Teil (11) besteht.

7. Linsenimplantat nach einem der vorangehenden Ansprüche, **dadurch gekennzeichnet, dass** eine distale Seite des zweiten Teils (11) in entspanntem Zustand gebogen ist zur Bildung der konvexen distalen Oberfläche (12a) des Linsenimplantats.

8. Linsenimplantat nach einem der vorangehenden Ansprüche, **dadurch gekennzeichnet, dass** der erste Teil (10) mindestens ein Material aufweist, das ausgewählt ist aus der Gruppe bestehend aus vernetzten Polysiloxanen, Hydrogelen und Collagen-Präparaten.

9. Linsenimplantat nach einem der vorangehenden Ansprüche, **dadurch gekennzeichnet, dass** der erste Teil besser verformbar ist als der zweite Teil, und insbesondere dass der erste Teil besser elastisch verformbar ist als der zweite Teil.

10. Linsenimplantat nach einem der vorangehenden Ansprüche, **dadurch gekennzeichnet, dass** der erste Teil ein Gel ist.

11. Linsenimplantat nach einem der vorangehenden Ansprüche, **dadurch gekennzeichnet, dass** der zweite Teil einen Beutel (11a) gefüllt mit einer Füllmasse (11b) aufweist.

12. Linsenimplantat nach einem der vorangehenden Ansprüche, **dadurch gekennzeichnet, dass** die proximale Oberfläche konvex ist mit einem Krümmungsradius zwischen 8.4 und 13.8 mm, und dass die distale Oberfläche konvex ist mit einem Krümmungsradius zwischen 4.6 und 7.5 mm, und dass das Linsenimplantat eine Dicke zwischen 2.8 und 5.5 mm aufweist.

13. Linsenimplantat nach einem der vorangehenden Ansprüche, **dadurch gekennzeichnet, dass** es derart dimensioniert und geformt ist, dass es die Dimensionen und Form einer auf die Ferne akkommodierten, natürlichen Linsenmasse besitzt.

14. Linsenimplantat nach einem der vorangehenden Ansprüche, **dadurch gekennzeichnet, dass** der erste Teil (10) ein Volumen grösser als das Volumen des zweiten Teils (11) aufweist.

15. Linsenimplantat nach einem der vorangehenden Ansprüche, **dadurch gekennzeichnet, dass** der erste Teil (10) einen grösseren Durchmesser als der zweite Teil (11) aufweist.

16. Bausatz zum Herstellen von Linsenimplantaten nach einem der vorangehenden Ansprüche, **gekennzeichnet durch** mindestens ein fliessfähiges Füllmaterial zum Bilden des ersten Teils (10) und eine Vielzahl von vorgeformten Linsenteilen zum Bilden des zweiten Teils (11), wobei die vorgeformten Linsenteile voneinander unterschiedliche Form und/oder Brechungsindizes besitzen.

17. Bausatz nach Anspruch 16, **dadurch gekennzeichnet, dass** die Linsenteile voneinander unterschiedliche Brechungsindizes besitzen.

18. Bausatz nach einem der Ansprüche 16 oder 17, **dadurch gekennzeichnet, dass** das Füllmaterial von einem fliessfähigen zu einem elastisch verformbaren Zustand aushärtbar ist.

19. Bausatz nach einem der Ansprüche 16 bis 18, **dadurch gekennzeichnet, dass** mindestens ein Teil der Linsenteile aus vorgeformten, füllbaren Beuteln (11a) bestehen.

## Claims

1. A lens implant for placement into the lens capsule (2) and for the replacement of the lens body (3) of an eye, which has the size and shape of a natural lens body, with a distal surface (12a) and a proximal surface (12b), wherein the distal surface (12a) is stronger curved than the proximal surface (12b), and with a first deformable part (10), which can be hardened from a pourable into an elastically deformable state, and a second part (11), which has a different refractive index and/or elastic deformability than the first part (10), wherein the second part (11) forms the distal surface (12a), **characterized in that** the first part (10) forms the proximal surface (12b).

2. The lens implant according to claim 1, **characterized in that** the second part has a different refractive index than the first part (11).

3. The lens implant according to one of the preceding claims, **characterized in that** the first part (10) and the second part (11) are abutting.

4. The lens implant according to one of the preceding claims, **characterized in that** the first part (10) can be hardened intra-ocularly from a pourable into an elastically deformable state.

5. The lens implant according to one of the preceding claims, **characterized in that** the second part (11) is elastically deformable for the purpose of a rolled-up or folded insertion into the lens capsule.

6. The lens implant according to one of the preceding claims, **characterized in that** the lens consists of the first part (10) and the second part (11).

7. The lens implant according to one of the preceding claims, **characterized in that** a distal side of the second part (11) is bent in a relaxed state, in order to shape the convex distal surface (12a) of the lens implant.

8. The lens implant according to one of the preceding claims, **characterized in that** the first part (10) comprises at least a material chosen from the group comprising cross-linked polysiloxanes, hydrogels and collagen-compounds.

9. The lens implant according to one of the preceding claims, **characterized in that** the first part is better deformable than the second part and in particular that the first part is better elastically deformable than the second part.

10. The lens implant according to one of the preceding claims, **characterized in that** the first part is a gel.

11. The lens implant according to one of the preceding claims, **characterized in that** the second part comprises a bag (11a) filled with a filling material (11b).

12. The lens implant according to one of the preceding claims, **characterized in that** the proximal surface is convex with a radius of curvature between 8.4 and 13.8 mm, and that the distal surface is convex with a radius of curvature between 4.6 and 7.5 mm, and the lens implant has a thickness between 2.8 and 5.5 mm.

13. The lens implant according to one of the preceding claims, **characterized in that** it is dimensioned and shaped in such a way, that it has the dimensions and shape of a natural lens body which is adapted to infinity.

14. The lens implant according to one of the preceding claims, **characterized in that** the first part (10) has a larger volume than the volume of the second part (11).

15. The lens implant according to one of the preceding claims, **characterized in that** the first part (10) has a larger diameter than the diameter of the second part (11).

16. A kit for producing lens implants according to one of the preceding claims, **characterized by** at least a pourable filling material for shaping the first part (10) and a plurality of preshaped lens parts for shaping the second part (11), wherein the preshaped lens parts have mutually different shapes and/or refractive indexes.

17. The kit according to claim 16, **characterized in that** the lens parts have mutually different refractive indexes.

18. The kit according to claim 16 or 17, **characterized in that** the filling material can be hardened from a pourable to an elastically deformable state.

19. The kit according to one of the claims 16 to 18, **characterized in that** at least a part of the lens parts consist of preshaped, fillable bags (11a).

## Revendications

1. Implant de lentille pour l'agencement dans la capsule de lentille (2) et pour le remplacement de la masse de lentille (3) d'un oeil, qui présente la grandeur et la forme d'une masse de lentille naturelle, avec une surface (12a) distale et une surface (12b) proximale, la surface (12a) distale étant incurvée davantage que la surface (12b) proximale, et une première partie (10) déformable, qui peut être durcie pour passer d'un état fluide à un état élastiquement déformable, et une seconde partie (11), qui présente un indice de réfraction différent de celui de la première partie (10) et/ou une déformabilité élastique différente de la première partie, la seconde partie (11) formant la surface (12a) distale, **caractérisé en ce que** la première partie (10) forme la surface (12b) proximale.

2. Implant de lentille selon la revendication 1, **caractérisé en ce que** la seconde partie présente un indice de réfraction différent de celui de la première partie (11).

3. Implant de lentille selon l'une quelconque des revendications précédentes, **caractérisé en ce que** la première partie (10) et la seconde partie (11) sont appuyées l'une contre l'autre.

4. Implant de lentille selon l'une quelconque des revendications précédentes, **caractérisé en ce que** la première partie (10) peut être durcie au plan intraoculaire en passant d'un état fluide à un état élastiquement déformable.

5. Implant de lentille selon l'une quelconque des revendications précédentes, **caractérisé en ce que** la seconde partie (11) est élastiquement déformable pour l'introduction enroulée ou pliée dans la capsule de lentille.

6. Implant de lentille selon l'une quelconque des revendications précédentes, **caractérisé en ce que** la lentille comprend la première partie (10) et la seconde partie (11).

7. Implant de lentille selon l'une quelconque des revendications précédentes, **caractérisé en ce qu'**un côté distal de la seconde partie (11) est plié dans l'état étendu pour former la surface (12a) distale convexe de l'implant de lentille.

8. Implant de lentille selon l'une quelconque des revendications précédentes, **caractérisé en ce que** la première partie (10) présente au moins un matériau qui est choisi dans le groupe composé de polysiloxanes réticulés, d'hydrogels et de préparations de collagène.

9. Implant de lentille selon l'une quelconque des revendications précédentes, **caractérisé en ce que** la première partie est plus déformable que la seconde partie, en particulier **en ce que** la première partie est plus déformable élastiquement que la seconde partie.

10. Implant de lentille selon l'une quelconque des revendications précédentes, **caractérisé en ce que** la première partie est un gel.

11. Implant de lentille selon l'une quelconque des revendications précédentes, **caractérisé en ce que** la seconde partie présente un sachet (11a) rempli d'une masse de remplissage (11b).

12. Implant de lentille selon l'une quelconque des revendications précédentes, **caractérisé en ce que** la surface proximale est convexe avec un rayon de courbure compris entre 8,4 et 13,8 mm, et **en ce que** la surface distale est convexe avec un rayon de courbure compris entre 4,6 et 7,5 mm, et **en ce que** l'implant de lentille présente une épaisseur comprise entre 2,8 et 5,5 mm.

13. Implant de lentille selon l'une quelconque des revendications précédentes, **caractérisé en ce qu'**il est dimensionné et formé de telle sorte qu'il présente les dimensions et la forme d'une masse de lentille naturelle et accommodée pour le lointain.

14. Implant de lentille selon l'une quelconque des revendications précédentes, **caractérisé en ce que** la première partie (10) présente un volume supérieur au volume de la seconde partie (11).

15. Implant de lentille selon l'une quelconque des revendications précédentes, **caractérisé en ce que** celui de la première partie (10) présente un diamètre plus grand que la seconde partie (11).

16. Kit pour fabriquer des implants de lentille selon l'une quelconque des revendications précédentes, **caractérisé par** au moins un matériau de remplissage fluide pour former la première partie (10) et une pluralité de parties de lentille préformées pour former la seconde partie (11), les parties de lentille préformées présentant des formes et/ou des indices de réfraction différents les uns des autres.

17. Kit selon la revendication 16, **caractérisé en ce que** les parties de lentille présentent des indices de réfraction différents les uns des autres.

18. Kit selon l'une quelconque des revendications 16 ou 17, **caractérisé en ce que** le matériau de remplissage peut être durci pour passer d'un état fluide à un état élastiquement déformable.

19. Kit selon l'une quelconque des revendications 16 à 18, **caractérisé en ce qu'**au moins une partie des parties de lentille est composée de sachets (11a) préformés et remplissables.
